# EUROPÄISCHE PATENTSCHRIFT

(11) **EP 3 730 011 B1**
(45) Veröffentlichungstag und Bekanntmachung des Hinweises auf die Patenterteilung: **28.05.2025**
(21) Anmeldenummer: 19170841.1
(22) Anmeldetag: 24.04.2019
(51) Int. Cl.: A47J 36/32, A47J 43/046

(54) **VERFAHREN ZUR ERNÄHRUNGSSTEUERUNG, KÜCHENGERÄT SOWIE SYSTEM ZUM ZUBEREITEN VON SPEISEN**
METHOD FOR NUTRITION CONTROL, KITCHEN APPLIANCE AND SYSTEM FOR PREPARING FOOD
PROCÉDÉ DE COMMANDE D'ALIMENTATION, APPAREIL DE CUISINE AINSI QUE SYSTÈME DE PRÉPARATION DES ALIMENTS

(43) Veröffentlichungstag der Anmeldung: 28.10.2020
(73) Patentinhaber: Vorwerk & Co. Interholding GmbH, 42270 Wuppertal (DE)
(72) Erfinder: Kraut-Reinkober, Stefan, 51375 Leverkusen (DE); Mosebach, Andrej, 44809 Bochum (DE); Pieper, Mirco, 42287 Wuppertal (DE); Stach, Christiane, 42477 Radevormwald (DE); Yan, Wenjie, 40476 Düsseldorf (DE); Werhahn, Sarah, 8038 Zürich (CH)
(74) Vertreter: Bals & Vogel Patentanwälte PartGmbB

(56) Entgegenhaltungen:
- EP-A1- 3 351 857
- WO-A1-2017/055317
- CN-A- 108 338 679
- DE-A1- 102017 112 855

## Beschreibung

Die Erfindung betrifft ein Verfahren zur Ernährungssteuerung, ein Küchengerät zum Zubereiten von Speisen sowie ein System zum Zubereiten von Speisen mit einem Küchengerät.

Küchengeräte zum Zubereiten von Speisen sind aus dem Stand der Technik bekannt. Dabei werden immer mehr Funktionen in Küchengeräte integriert, um einem Benutzer das Zubereiten von Speisen zu erleichtern. So können die Küchengeräte teilweise Rezepte selbstständig abarbeiten, indem Zutaten im Küchengerät automatisch erhitzt werden, zerkleinert werden oder dergleichen.

Darüber hinaus wird immer häufiger, z.B. über Fitnessarmbänder in die täglichen Abläufe von Benutzern eingegriffen, um diese zu optimieren und dadurch die Gesundheit und/oder das Wohlbefinden der Benutzer zu verbessern. Aus dem Ernährungssektor ist dies bspw. aus der US 2016/0048720 A1 bekannt, wobei einem Benutzer einer Küchenmaschine Eckdaten von benutzten Nahrungsmitteln angezeigt werden, die auch Gesundheitsdaten enthalten. Dadurch kann der Benutzer schnell erkennen, ob die Nahrungsmittel in seinen aktuellen Ernährungsplan hineinpassen. Nachteilig ist jedoch, dass der Benutzer hierbei keine Unterstützung bei der Verarbeitung der angezeigten Daten erhält. Vielmehr ist er selbst darauf angewiesen, die Daten für sich zu verwerten. So ist es z.B. wünschenswert einem Benutzer, der weniger kreativ bei der Auswahl seiner Speisen ist, mit Bezug auf seine Gesundheit, Unterstützung automatisch anzubieten.

Aus dem Dokument DE 10 2017 112 855 A1 sind ferner ein Küchengerät und ein Verfahren zur Verwertung von Informationen über Gesundheitseigenschaften bei der Bedienung eines Küchengeräts bekannt.

Es ist eine Aufgabe der vorliegenden Erfindung voranstehende aus dem Stand der Technik bekannte Nachteile zumindest teilweise zu beheben. Insbesondere ist es eine Aufgabe der vorliegenden Erfindung zumindest einen Benutzer eines Küchengerätes bei der Wahl seiner Ernährung im Hinblick auf seine Gesundheit zu unterstützen.

Die voranstehende Aufgabe wird gelöst durch ein Verfahren mit den Merkmalen des unabhängigen Verfahrensanspruchs, ein Küchengerät mit den Merkmalen des unabhängigen Vorrichtungsanspruchs und ein System mit den Merkmalen des unabhängigen Systemanspruchs. Weitere Merkmale und Details der Erfindung ergeben sich aus den Unteransprüchen, der Beschreibung und den Zeichnungen. Dabei gelten Merkmale und Details, die im Zusammenhang mit dem erfindungsgemäßen Verfahren beschrieben worden sind, selbstverständlich auch im Zusammenhang mit dem erfindungsgemäßen Küchengerät und/oder dem erfindungsgemäßen System und jeweils umgekehrt, sodass bzgl. der Offenbarung zu den einzelnen Erfindungsaspekten stets wechselseitig Bezug genommen wird bzw. werden kann.

Erfindungsgemäß umfasst das Verfahren zur Ernährungssteuerung mit einem Küchengerät zum zumindest teilweise automatisierten Abarbeiten eines Rezeptes die folgenden Schritte:
- Erhalt von nutzerspezifischen Gesundheitsdaten, die einem Benutzerprofil zuordbar sind,
- Erstellen einer Auswertung der Gesundheitsdaten für das Benutzerprofil durch eine Recheneinheit,
- Bestimmen einer Rezeptempfehlung auf Grundlage der Auswertung der Gesundheitsdaten,
- Ausgabe der Rezeptempfehlung an einer Benutzerschnittstelle des Küchengerätes.

Vorzugsweise findet eine zeitliche Abarbeitung der Verfahrensschritte in der aufgeführten Reihenfolge statt.

Das Küchengerät kann insbesondere auch als Küchenmaschine, Kochmixer oder intelligenter Ofen, bezeichnet werden. Zum zumindest teilweise automatisierten Abarbeiten des Rezeptes kann vorgesehen sein, dass das Küchengerät ein Rührwerk zum Zerkleinern von Zutaten des Rezeptes und/oder ein Heizelement zum Erhitzen von Zutaten des Rezeptes aufweist. Darüber hinaus sind ferner weitere Funktionseinheiten zum automatischen Durchführen von Zubereitungsschritten denkbar. Der Erhalt der nutzerspezifischen Gesundheitsdaten, die dem Benutzerprofil zuordbar sind, kann zumindest teilweise über eine Kommunikationsschnittstelle des Küchengerätes erfolgen. So können die Gesundheitsdaten bereits mit dem Benutzerprofil verknüpft sein und z.B. auf einem Server oder auf dem Küchengerät selbst hinterlegt sein. Ferner ist es denkbar, dass die Gesundheitsdaten dem Benutzerprofil nach dem Erhalt zugeordnet werden. So kann vorgesehen sein, dass ein Benutzer die Gesundheitsdaten über die Küchenmaschine oder ein Nutzergerät eingibt und diese dadurch dem Benutzerprofil zuordnet. Die Auswertung der Gesundheitsdaten kann insbesondere eine Benutzerklassifikation umfassen. So ist es denkbar, dass einem Benutzer bestimmte Klassifikationsdaten im Rahmen der Auswertung zugeordnet werden, wie z.B. "Diabetiker, Allergiker, Sportler und/oder dergleichen". Weiterhin ist es denkbar, dass bei der Auswertung bestimmte Nahrungsparameter erstellt werden, die bei der Rezeptempfehlung berücksichtigt werden, um die Gesundheit des Benutzers zu verbessern. Ergibt die Auswertung der Gesundheitsdaten bspw., dass der Benutzer einen Eisenmangel aufweist, kann dies zu besonders eisenreichen Rezeptempfehlungen führen. Die Rezeptempfehlung kann insbesondere einen konkreten Rezeptvorschlag, eine Anpassung und/oder eine Änderung eines vorliegenden Rezeptes und/oder einen Vorschlag für eine Rezeptklassifikation umfassen. Auch ist es denkbar, dass bewusst gewisse Rezepte mit Zutaten, die eine Allergie und/oder Unverträglichkeit beim Benutzer auslösen können, vermieden werden oder Vergleichsvorschläge für Rezepte ohne diese allergieauslösenden Zutaten angeboten werden. Der Rezeptvorschlag kann insbesondere ein auf dem Küchengerät ausführbares Rezept umfassen. Die Rezeptklassifikation kann z.B. "low-carb" Rezepte, vegetarische Rezepte und/oder dergleichen umfassen. Insbesondere kann das Erstellen der Auswertung und das Bestimmen der Rezeptempfehlung gleichzeitig durchgeführt werden, wobei z.B. die Auswertung unmittelbar in Hinblick auf die Rezeptempfehlung durchgeführt werden kann. Zur Ausgabe der Rezeptempfehlung an der Benutzerschnittstelle des Küchengerätes kann vorgesehen sein, dass die Benutzerschnittstelle ein Display aufweist, an welchem die Rezeptempfehlung angezeigt werden kann. Insbesondere kann vorgesehen sein, dass der Benutzer über die Benutzerschnittstelle mit dem Küchengerät interagieren kann, um auf die Rezeptempfehlung zu reagieren, indem er bspw. die Rezeptempfehlung annimmt oder ablehnt. Weiterhin kann vorgesehen sein, dass die Rezeptempfehlung zusätzlich an einem weiteren Display, z.B. über eine App auf einem Nutzergerät, erfolgt. Ein Nutzergerät kann bspw. ein Smartphone, Tablet, Computer und/oder dergleichen umfassen.

Somit kann in komfortabler Art und Weise die Ernährung des Benutzers positiv beeinflusst werden. Dadurch dass die Gesundheitsdaten automatisch ausgewertet werden und zu einer Rezeptempfehlung führen, können dem Benutzer auch solche Rezepte vorgeschlagen werden, welche ihm zuvor unbekannt waren oder welche in Vergessenheit geraten waren. Durch die Ausgabe der Rezeptempfehlung einer Benutzerschnittstelle des Küchengerätes ist zum einen die Möglichkeit geschaffen, dass der Benutzer direkt am Küchengerät die Schritte ausführen kann und somit komfortabel auf das Rezept bzw. auf die Rezeptempfehlung zugreifen kann. Zum anderen kann dadurch eine Nähe zum eigentlichen Kochvorgang geschaffen sein, durch welche eine Hemmschwelle sinken kann, die Rezeptempfehlung tatsächlich zu berücksichtigen und das zugrundeliegende Rezept umzusetzen. An der Stelle der Auswertung der Gesundheitsdaten und/oder das Bestimmen der Rezeptempfehlung kann dies vorzugsweise auf dem Küchengerät selbst, einem Server und/oder einem Nutzergerät des Benutzers durchgeführt werden. Werden die Schritte auf dem Küchengerät selbst durchgeführt, kann dieses autark zu einer Internetverbindung sein, d.h. insbesondere unabhängig von einer Internetverbindung betreibbar sein. Durch die Ausführung der Schritte auf einem Server kann der Vorteil erzielt werden, dass die Rechenkapazität vom Küchengerät auslagerbar sein kann, sodass eine Recheneinheit des Küchengerätes klein dimensioniert sein kann. Ferner können weitere Daten, insbesondere im Rahmen einer Big-Data-Analyse in die Auswertung und/oder die Rezeptempfehlung einfließen. Durch die Anbindung eines Nutzergerätes kann insbesondere ein Fernzugriff auf das Benutzerprofil ermöglicht sein, sodass der Benutzer das Benutzerprofil komfortabel verwalten kann.

Im Rahmen der Erfindung kann ferner vorgesehen sein, dass die Gesundheitsdaten Vitaldaten eines Benutzers umfassen. Die Vitaldaten können insbesondere Werte zu Blutzucker, Gewicht, Blutdruck, Unverträglichkeiten, Allergien von Stoffen und/oder dergleichen umfassen. Ferner ist es denkbar, dass die Vitalwerte weitere Eigenschaften des Benutzers umfassen, wie z.B. ein Alter und/oder dergleichen. Ferner können die Vitaldaten Aktivitätsdaten umfassen, wobei die Aktivitätsdaten z.B. aus einem Fitnessarmband, aus Kalenderdaten des Benutzers und/oder weiteren Datenquellen erhalten werden können. Insbesondere kann es sich bei den Vitaldaten somit um statische oder sich dynamisch verändernde Gesundheitswerte des Benutzers handeln, welche Aufschluss über den aktuellen Gesundheitszustand des Benutzers geben können. Dadurch kann die Rezeptempfehlung individuell auf die Bedürfnisse des Benutzers angepasst werden, um einen positiven Einfluss auf dessen Ernährung zu verstärken.

Bei einem erfindungsgemäßen Verfahren ist ferner vorgesehen, dass beim Erstellen der Auswertung eine Benutzerreaktion und eine Vitalveränderung auf eine frühere Rezeptempfehlung berücksichtigt wird. Bei einem erfindungsgemäßen Verfahren kann ferner vorgesehen sein, dass das Erstellen der Auswertung einen Vergleich der Gesundheitsdaten mit Referenzdaten umfasst und/oder dass beim Erstellen der Auswertung eine Benutzerreaktion und/oder eine Vitalveränderung auf eine frühere Aktivitätsempfehlung berücksichtigt wird. Dadurch werden für die Auswertung Randbedingungen in Form der Benutzerreaktion und der Vitalveränderung und vorzugsweise der Referenzdaten gesetzt. Ferner ist es denkbar, dass die Vitalveränderung und/oder die Benutzerreaktion können beim Bestimmen der Rezeptempfehlung berücksichtigt werden. Die Referenzdaten können bspw. ideale Parameter sein, die zu den Gesundheitsdaten korrespondieren. So kann im Rahmen der Auswertung bspw. vorgesehen sein, dass ein Blutzuckerwert des Benutzers mit einem Blutzuckerwert verglichen wird, der ideal für einen Benutzer in dessen Alterskategorie und/oder dessen Gewichtskategorie ist. Die Benutzerreaktion kann bspw. umfassen, ob der Benutzer die Rezeptempfehlung annimmt und das dahinterliegende Rezept tatsächlich ausführt. Die Vitalveränderung kann z.B. eine Veränderung der Gesundheitsdaten nach Ausführung der Rezeptempfehlung umfassen. Weiterhin kann die Vitalreaktion auf eine frühere Aktivitätsempfehlung festgestellt und berücksichtigt werden. Wird bspw. festgestellt, dass der Benutzer an Gewicht zunimmt, obwohl dies nicht gewünscht war, können somit Rückschlüsse für zukünftige Rezeptempfehlungen gezogen werden. Dadurch kann sich ein Lerneffekt beim Ausführen des Verfahrens ergeben, sodass die Rezeptempfehlung insbesondere nach einer Anlernphase für den Benutzer eine hohe Genauigkeit bei der Auswahl der Rezepte für die Rezeptempfehlung aufweist. Ferner kann anhand der Benutzerreaktion auch ein Geschmack des Benutzers berücksichtigt werden. Lehnt der Benutzer bspw. Rezeptempfehlungen mit einer bestimmten Zutat mehrfach ab, kann daraus geschlossen werden, dass dies dem Geschmack des Benutzers nicht entspricht und folglich zukünftig Rezepte mit derartigen Zutaten geringer priorisiert werden oder vermieden werden. Insbesondere kann die Benutzerreaktion und/oder die Vitalveränderung den Referenzdaten hinzugefügt werden, um für erneute Auswertungen zur Verfügung zu stehen.

Ferner kann bei einem erfindungsgemäßen Verfahren vorgesehen sein, dass das Verfahren folgenden Schritt umfasst:
- Auswerten von Historiendaten zuvor durchgeführter Kochvorgänge und/oder zuvor verwendeter Lebensmittel,
wobei die Historiendaten beim Erstellen der Rezeptempfehlung berücksichtigt werden. Die Historiendaten können z.B. dadurch ermittelt werden, dass zuvor ausgeführte Rezepte aufgezeichnet bzw. abgespeichert werden. Zuvor verwendete Lebensmittel können z.B. durch eine Kamera, einen Barcodescanner, einen Ultraschallsensor, einen Radarsensor, insbesondere einen Kurzstreckenradarsensor, eine Waage, vorzugsweise mit zumindest drei Wägezellen, und/oder weitere Sensoriken erkannt werden. Ferner ist es denkbar, dass die zuvor durchgeführten Kochvorgänge und/oder die zuvor verwendeten Lebensmittel anhand von Daten von Lieferdiensten ausgewertet werden. Dadurch können auch solche Historiendaten verwendet werden, die von Drittanbietern bereitgestellt werden, um die Auswertung der Gesundheitsdaten und/oder die Rezeptempfehlung zu verbessern.

Im Rahmen der Erfindung ist es ferner denkbar, dass das Verfahren folgenden Schritt umfasst:
- Ermitteln von Positionsinformationen des Küchengerätes und/oder eines Benutzers, wobei die Positionsinformationen beim Erstellen der Rezeptempfehlung berücksichtigt werden. Die Positionsinformationen können insbesondere durch einen GPS-Sensor im Küchengerät und/oder in einem Nutzergerät ermittelt werden. Ferner ist es denkbar, dass die Positionsinformationen durch einen Netzwerkstandort des Küchengerätes und/oder des Benutzers, insbesondere anhand einer IP- Adresse und/oder anhand von Mobilfunkdaten, ermittelt werden. Die Positionsinformationen können insbesondere den Standort des Küchengerätes und/oder Aktivitätsdaten des Benutzers umfassen. So kann über die Positionsinformationen z.B. festgestellt werden, ob der Benutzer sportlich aktiv war oder bestimmte Restaurants besucht hat. Dies kann entsprechend mit den Gesundheitsdaten verarbeitet werden und zu einer verbesserten Rezeptempfehlung führen. Über den Standort des Küchengerätes kann z.B. festgestellt werden, in welcher Region sich das Küchengerät befindet, sodass Rückschlüsse auf allgemeine Befindlichkeiten in der Bevölkerung dieser Region getätigt werden können, um die Rezeptempfehlung zu verbessern.

Vorzugsweise kann bei einem erfindungsgemäßen Verfahren das Verfahren folgenden Schritt umfassen:
- Zumindest teilweise automatisiertes Abarbeiten der Rezeptempfehlung durch das Küchengerät.

Somit kann die Hemmschwelle des Benutzers gesenkt werden, der Rezeptempfehlung zu folgen, wenn das Küchengerät die Rezeptempfehlung automatisch startet und/oder zumindest teilweise automatisch ausführen kann. Dadurch kann der Benutzer bspw. motiviert werden neue Rezepte im Rahmen der Rezeptempfehlung auszuprobieren, wenn der Zubereitungsvorgang an sich vereinfacht ist. Ferner kann die Rezeptempfehlung dadurch einfacher in den Alltag des Benutzers eingebunden werden.

Weiterhin kann bei einem erfindungsgemäßen Verfahren vorgesehen sein, dass das Bestimmen der Rezeptempfehlung zumindest einen der folgenden Schritte umfasst:
- Auswahl eines Rezeptes aus einer Rezeptdatenbank anhand der Auswertung der Gesundheitsdaten, eines Benutzerziels und/oder einer Vitalveränderung,
- Generierung eines Rezeptes anhand der Auswertung der Gesundheitsdaten, eines Benutzerziels und/oder einer Vitalveränderung, insbesondere wobei die Historiendaten bei der Generierung des Rezeptes berücksichtigt werden.

Bei der Vitalveränderung kann es sich insbesondere um eine Vitalveränderung des Benutzers auf eine frühere Rezeptempfehlung handeln. Das Benutzerziel kann vorzugsweise manuell vorgegeben sein. Die Generierung des Rezeptes kann insbesondere eine Modifikation eines bestehenden Rezeptes umfassen. Vorzugsweise kann bei der Generierung eine individuelle Menüzusammenstellung anhand der Bedürfnisse des Benutzers erfolgen. Die Historiendaten können insbesondere für die Geschmacksbestimmung herangezogen werden, wobei vorzugsweise bei der Generierung des Rezeptes ein Verhaltensmuster des Benutzers anhand der Historiendaten analysiert wird, um dessen Präferenzen zu erkennen. Wird das Rezept aus der Rezeptdatenbank anhand der Auswertung der Gesundheitsdaten, des Benutzerziels und/oder des Vitalveränderung ausgewählt, kann ferner auf bestehende Rezepte zurückgegriffen werden, die sich z.B. bei anderen Benutzern bereits als vorteilhaft herausgestellt haben. Dazu kann vorgesehen sein, dass die Rezepte in der Rezeptdatenbank anhand von Schlagwörtern ausgewählt werden.

Im Rahmen der Erfindung kann ferner vorgesehen sein, dass ein externes Empfangen der Gesundheitsdaten durch die Geräteschnittstelle möglich ist und ein externes Versenden der Gesundheitsdaten zumindest teilweise verhindert ist, insbesondere wobei das externe Versenden der Gesundheitsdaten durch den Benutzer freischaltbar oder selektiv freischaltbar ist. Unter dem externen Versenden der Gesundheitsdaten kann verstanden werden, dass die Gesundheitsdaten vom Küchengerät und/oder von einem Server aus versendet werden und von Stellen, insbesondere externen Dienstleistern, empfangen werden, die nicht unmittelbar dem Benutzerprofil zugeordnet sind. Dabei kann es sich z.B. um Ärzte, ein Fitnessstudio, Lieferdienste, eine Versicherung und/oder weitere externe Dienstleister handeln. Ist lediglich das Empfangen, nicht aber das Versenden möglich, können Daten von den externen Stellen gewonnen werden, die die Rezeptempfehlung verbessern können und weiteren Aufschluss über den Gesundheitszustand des Benutzers geben können. Gleichzeitig kann dabei eine Datensicherheit gegeben sein, wenn diese Daten nicht durch die externen Stellen ausgelesen werden können. Insbesondere kann durch die Freischaltung durch den Benutzer ermöglicht sein, dass der Benutzer selbstständig seine Daten verwalten kann und z.B. nur solchen Diensten Zugriff auf die Daten erlauben kann, denen er vertraut. Unter der selektiven Freischaltung kann verstanden werden, dass lediglich bestimmte Daten mit externen Diensten geteilt werden und/oder Daten lediglich an bestimmte Dienste gesendet werden können. Da es sich bei Gesundheitsdaten um besonders sensible Daten handelt, kann eine erhöhte Datensicherheit auch zu einer verbesserten Akzeptanz des gesamten Verfahrens mit der Verarbeitung von Gesundheitsdaten führen.

Vorzugsweise kann bei einem erfindungsgemäßen Verfahren vorgesehen sein, dass die Gesundheitsdaten Aktivitätsdaten eines Benutzers umfassen und der Erhalt der Gesundheitsdaten folgenden Schritt umfasst:
- Erfassen von Aktivitätsdaten.

Aktivitätsdaten können insbesondere Auswärtsaktivitäten des Benutzers umfassen wie z.B. Restaurantbesuche, Essen bei Freunden und Verwandten und/oder dergleichen. Dadurch können weitere Daten gewonnen werden und somit die Rezeptempfehlung in Bezug auf den Benutzer verbessert sein. Weiterhin können die Aktivitätsdaten auch sportliche Aktivitäten des Benutzers umfassen, sodass z.B. zur Unterstützung eines Muskelaufbaus des Benutzers in Abhängigkeit von der sportlichen Aktivität die Rezeptempfehlung proteinreiche Nahrung bevorzugt. Insbesondere können die Aktivitätsdaten zur Gewichtung weiterer Gesundheitsdaten eingesetzt werden.

Ferner ist es bei einem erfindungsgemäßen Verfahren denkbar, dass der Erhalt der Gesundheitsdaten über eine Sensoreinheit erfolgt, insbesondere wobei die Sensoreinheit ein Nutzergerät und/oder einen der folgenden Sensoren umfasst:
- Kamera,
- GPS-Sensor,
- Waage,
- Temperatursensor (insbesondere berührungslos, z.B. Infrarot-Messung)
- Herzfrequenzsensor,
- Blutzuckersensor,
- Blutdrucksensor,
- Pulssensor,
- Körperfettsensor.

Die Sensoreinheit kann dazu z.B. an einem Fitnessarmband, einer Smartwatch, einem Smartphone und/oder dergleichen vorgesehen sein. Insbesondere können dadurch tatsächliche Messwerte, insbesondere auf den aktuellen Gesundheitszustand des Benutzers bezogen, zur Erstellung der Rezeptempfehlung berücksichtigt werden. Es ist bspw. nicht notwendig, dass der Benutzer die Gesundheitsdaten manuell eingibt, sondern diese können insbesondere in Echtzeit gewonnen werden. Dadurch kann z.B. die Häufigkeit der Erhebung der Gesundheitsdaten vergrößert werden, und damit die Genauigkeit der Auswertung verbessert werden. Ferner kann durch einen nutzernahen Sensor der Bedienkomfort verbessert sein. Insbesondere können auch weitere Daten, wie z.B. Positionsinformationen, durch die Sensoreinheit erfasst werden.

Weiterhin ist es bei einem erfindungsgemäßen Verfahren denkbar, dass das Verfahren folgenden Schritt umfasst:
- Erhalt eines Benutzerziels, das dem Benutzerprofil zuordbar ist,
wobei das Benutzerziel beim Bestimmen der Rezeptempfehlung berücksichtigt wird. Das Benutzerziel kann z.B. das Abnehmen bis zu einem bestimmten Gewicht umfassen, die Teilnahme an einer Sportveranstaltung und/oder einen bestimmten Fitnesslevel. Dadurch kann eine Motivation des Benutzers verbessert werden, der Rezeptempfehlung zu folgen, wenn der Ehrgeiz des Benutzers angeregt wird. Weiterhin kann die Rezeptempfehlung dadurch dahingehend verbessert werden, dass diese auf ein individuelles, subjektives Bedürfnis des Benutzers zugeschnitten wird und somit die Gesundheit des Benutzers in einem bestimmten Aspekt verbessert werden kann. Insbesondere kann das Benutzerziel und/oder ein Fortschritt an der Benutzerschnittstelle bereitstellbar, insbesondere anzeigbar, sein.

Ferner kann bei einem erfindungsgemäßen Verfahren vorgesehen sein, dass in Abhängigkeit von den Gesundheitsdaten, den Positionsinformationen und/oder dem Benutzerziel eine Rezeptfolge bestimmt wird. Vorzugsweise umfasst die Rezeptfolge mehrere Rezeptempfehlungen. Die Rezeptfolge kann insbesondere eine Art Rezept-Playlist umfassen, bei welcher sich der Benutzer von möglicherweise unbekannten Rezepten überraschen lassen kann. Die Reihenfolge der Rezeptfolge kann dabei variabel oder vorgegeben sein. Insbesondere kann die Rezeptfolge dem Benutzer an der Benutzerschnittstelle und/oder einem Nutzergerät des Benutzers zur Verfügung gestellt werden. Somit können mehrere Rezeptempfehlungen für zukünftige Speisen des Benutzers gegeben werden. Dadurch kann das Ernährungsverhalten des Benutzers langfristig verbessert werden und gleichzeitig dem Benutzer der Komfort ermöglicht werden, dass dieser die Rezeptempfehlungen bereits für einige Tage im Voraus kennt und somit seinen Einkauf entsprechend anpassen kann.

Weiterhin kann bei erfindungsgemäßen Verfahren vorgesehen sein, dass die Rezeptfolge in Abhängigkeit von einer Benutzerreaktion und/oder einer Vitalveränderung auf eine Rezeptempfehlung, insbesondere die bestimmte und/oder eine frühere Rezeptempfehlung, der Rezeptfolge modifiziert wird. Wird bspw. festgestellt, dass der Benutzer eine Rezeptempfehlung der Rezeptfolge ablehnt oder sich dessen Gesundheitsdaten nicht verbessern oder sogar verschlechtern, kann die Rezeptfolge entsprechend angepasst werden. Weiterhin können Rezeptempfehlungen innerhalb der Rezeptfolge ersetzt und/oder modifiziert werden. Insbesondere kann somit eine Adaptierung erreicht werden, um das Benutzerziel möglichst effizient zu erreichen.

Weiterhin ist es bei einem erfindungsgemäßen Verfahren denkbar, dass dem Benutzerprofil eine Benutzergruppe, insbesondere ein Haushalt, zugeordnet wird. Somit ist es bspw. nicht notwendig, dass jeder Benutzer ein eigenes Benutzerprofil erstellt, sondern ein Haushalt kann auch in einer Benutzergruppe zusammengefasst werden. Somit können die einzelnen Benutzer innerhalb des Benutzerprofils komfortabel verwaltet werden und deren Gewohnheiten und/oder Benutzerziele aufeinander abgestimmt werden. Wenn z.B. die Benutzergruppe eine Familie ist, kann dadurch die Gesundheit aller oder möglichst vieler Mitglieder der Benutzergruppe verbessert werden.

Im Rahmen der Erfindung ist es ferner denkbar, dass das Verfahren folgende Schritte umfasst:
- Bestimmen einer Aktivitätsempfehlung auf Grundlage der Auswertung der Gesundheitsdaten,
- Ausgabe der Aktivitätsempfehlung, insbesondere an der Benutzerschnittstelle des Küchengerätes.

Durch die Aktivitätsempfehlung kann die Gesundheit des Benutzers dadurch verbessert werden, dass insbesondere flankierend zu der Rezeptempfehlung auch ein Verhalten des Benutzers optimiert werden kann. Die Ausgabe der Aktivitätsempfehlung kann an der Benutzerschnittstelle des Küchengerätes und/oder an einem Nutzergerät des Benutzers erfolgen. Ist z.B. im Rahmen des Benutzerziels vorgegeben, dass der Benutzer an einem Marathon teilnehmen möchte, kann durch die Aktivitätsempfehlung z.B. ein Trainingsplan vorgegeben sein und durch die Rezeptempfehlung gleichzeitig ein auf diesen Trainingsplan abgestimmtes Ernährungsverhalten des Benutzers verbessert werden. Damit kann schließlich die Gesundheit des Benutzers insgesamt verbessert werden.

Gemäß einem weiteren Aspekt der Erfindung ist ein Küchengerät nach Anspruch 12 vorgesehen.

Unter dem Initiieren des Verfahrens kann vorzugsweise verstanden werden, dass das Verfahren durch das integrierte Steuergerät des Küchengerätes auslösbar bzw. in Gang setzbar ist. So ist es denkbar, dass das integrierte Steuergerät dazu ausgebildet ist, mit einem Server in Kommunikationsverbindung zu treten und den Server derart anzusteuern, dass das Verfahren zumindest teilweise oder vollständig auf dem Server durchgeführt wird. Über die Benutzerschnittstelle können schließlich die Rezeptempfehlungen oder die Rezeptempfehlung bereitgestellt werden. Die Benutzerschnittstelle kann vorzugsweise ein Display und/oder ein Bedienelement aufweisen, wobei das Display vorzugsweise ein Touchdisplay sein kann. Dadurch kann ein Benutzer in komfortabler Art und Weise mit dem Küchengerät interagieren und Steuerbefehle an das integrierte Steuergerät des Küchengerätes übermitteln. Unter dem integrierten Steuergerät kann vorzugsweise ein internes Steuergerät des Küchengerätes verstanden werden, welches fest mit dem Küchengerät verbaut ist. Die Funktionseinheit zum Bearbeiten von Zutaten kann dazu ausgebildet sein, dass zumindest teilweise automatisierte Abarbeiten eines Rezeptes zu ermöglichen, indem bestimmte Rezeptschritte wie z.B. das Erhitzen von Zutaten, das Zerkleinern von Zutaten und/oder dergleichen in Abhängigkeit von einer Ansteuerung durch das integrierte Steuergerät durchzuführen. Dazu kann die Funktionseinheit z.B. ein Rührwerk und/oder ein Heizelement aufweisen. Vorzugsweise kann durch das integrierte Steuergerät das Verfahren insbesondere vollständig am Küchengerät durchführbar sein.

Dadurch ist es bspw. nicht notwendig das Küchengerät mit dem Internet zu verbinden, um auf einen Server zugreifen zu können und dessen Rechenleistung zu nutzen. Ferner kann dadurch ermöglicht sein, dass ein Benutzer unabhängig von der Internetverbindung das Küchengerät insbesondere autark betreiben kann, z.B. direkt nach dem Kauf. Somit bringt ein erfindungsgemäßes Küchengerät die gleichen Vorteile mit sich, wie sie bereits ausführlich mit Bezug auf ein erfindungsgemäßes Verfahren beschrieben worden sind.

Gemäß einem weiteren Aspekt der Erfindung ist ein System nach Anspruch 13 vorgesehen.

Vorzugsweise kann zusätzlich vorgesehen sein, dass die Recheneinheit dazu ausgebildet ist einem Nutzergerät die Rezeptempfehlung über eine Kommunikationsverbindung zur Verfügung zu stellen. Dadurch kann sich ein Fernzugriff des Benutzers auf die Rezeptempfehlung ermöglichen. Insbesondere kann die Rezeptempfehlung auf einem externen Display des Nutzergerätes und/oder durch Aufrufen einer App auf dem Nutzergerät abrufbar sein. Ein erfindungsgemäßes Verfahren ist durch die Recheneinheit ausführbar. Somit bringt ein erfindungsgemäßes System die gleichen Vorteile mit sich, wie sie bereits ausführlich mit Bezug auf ein erfindungsgemäßes Küchengerät und/oder ein erfindungsgemäßes Verfahren beschrieben worden sind.

Vorzugsweise kann bei einem erfindungsgemäßen System vorgesehen sein, dass die Recheneinheit und/oder die Speichereinheit zur Erhebung von nutzerspezifischen Gesundheitsdaten mit einem weiteren Küchengerät und/oder einem Nutzergerät in Kommunikationsverbindung bringbar ist. Insbesondere ist es denkbar, dass es sich bei der Speichereinheit um einen Speicherchip handelt, der mit dem Küchengerät, insbesondere über die Geräteschnittstelle, verbindbar ist. Das Nutzergerät kann vorzugsweise ein Fitnessarmband, ein Smartphone, ein Tablet und/oder dergleichen umfassen. Insbesondere kann durch die Kommunikationsverbindung auch ermöglicht sein, dass ein Fernzugriff auf die Recheneinheit und/oder eine Ferndarstellung der Recheneinheit über ein Display und/oder eine App des Nutzergerätes dem Benutzer ermöglicht ist. Zur Kommunikationsverbindung kann das System, insbesondere das Küchengerät, eine Geräteschnittstelle aufweisen, durch welche das Küchengerät mit dem weiteren Küchengerät und/oder dem Nutzergerät in Kommunikationsverbindung bringbar ist. Durch die Erhebung nutzerspezifischer Gesundheitsdaten auf einem weiteren Küchengerät und/oder einem Nutzergerät ist es einem Benutzer auch dann möglich, die Gesundheitsdaten zur Verarbeitung entsprechend eines erfindungsgemäßen Verfahrens zu übermitteln, wenn er z.B. unterwegs oder an einem weiteren Küchengerät kocht. So ist es z.B. denkbar, dass das weitere Küchengerät an einer Arbeitsstelle des Benutzers aufgestellt ist, sodass die nutzerspezifischen Gesundheitsdaten sowohl von Zuhause als auch auf der Arbeit erhoben werden können. Dadurch kann die Ernährung des Benutzers zu einem hohen Grad umfassend abgebildet sein. Das Nutzergerät kann dies ebenso ermöglichen. Ferner kann durch das Nutzergerät auch eine Kontrolle der Ernährungsdaten bzw. der Gesundheitsdaten von unterwegs ermöglicht sein.

Vorzugsweise ist bei einem erfindungsgemäßen System ein Server vorgesehen, der die Recheneinheit und/oder die Speichereinheit umfasst. Alternativ kann die Recheneinheit und/oder die Speichereinheit Teil des integrierten Steuergerätes des Küchengerätes sein. Ist die Recheneinheit und/oder die Steuereinheit zumindest teilweise auf den Server ausgelagert, kann eine zentrale Rechenkapazität des Servers genutzt werden und das Küchengerät kann in der Rechenleistung kleiner dimensioniert sein. Dadurch können sich Kostenvorteile des Küchengerätes ergeben und ein Zugriff auf weitere Daten, insbesondere auf weitere Datenbanken, kann durch den Server erleichtert sein.

Weiterhin kann bei einem erfindungsgemäßen System vorgesehen sein, dass das Küchengerät eine Geräteschnittstelle aufweist, durch welche das Küchengerät mit der Recheneinheit und/oder der Speichereinheit, insbesondere zum Zugriff auf eine Rezeptdatenbank, in Kommunikationsverbindung bringbar ist. Insbesondere kann dazu auch der Server eine entsprechende Schnittstelle aufweisen. Die Geräteschnittstelle kann eine kabelgebundene oder eine kabellose Geräteschnittstelle sein, insbesondere ist es denkbar, dass das Küchengerät über ein Netzwerk, insbesondere eine Ethernet-Verbindung, eine WLAN- Verbindung, eine Mobilfunkverbindung und/oder dergleichen mit der Recheneinheit und/oder der Speichereinheit in Kommunikationsverbindung bringbar ist. Weiterhin ist es denkbar, dass die Recheneinheit und/oder die Speichereinheit über eine lokale Verbindung mit dem Küchengerät in Kommunikationsverbindung bringbar ist. So kann es sich bei der Geräteschnittstelle z.B. um eine USB-Schnittstelle, eine Bluetooth-Schnittstelle und/oder dergleichen handeln. Dadurch kann in einfacher Art und Weise eine entsprechende Kommunikationsverbindung ermöglicht sein.

Weitere, die Erfindung verbessernde Maßnahmen ergeben sich aus der nachfolgenden Beschreibung zu einigen Ausführungsbeispielen der Erfindung, welche in den Figuren schematisch dargestellt sind. Dabei ist es zu beachten, dass die Figuren nur beschreibenden Charakter haben und nicht dazu gedacht sind, die Erfindung in irgendeiner Form einzuschränken. Es zeigen schematisch:
- Figur 1: ein erfindungsgemäßes System mit einem erfindungsgemäßen Küchengerät zum Ausführen eines erfindungsgemäßen Verfahrens in einem ersten Ausführungsbeispiel,
- Figur 2: das Verfahren des ersten Ausführungsbeispiels in schematischer Darstellung der Verfahrensschritte,
- Figur 3: eine Auswertung von Gesundheitsdaten beim Verfahren des ersten Ausführungsbeispiels,
- Figur 4: ein Benutzerprofil beim erfindungsgemäßen Verfahren des ersten Ausführungsbeispiels,
- Figur 5: ein Zusammenspiel mit einem externen Dienstleister beim Verfahren des ersten Ausführungsbeispiels,
- Figur 6: ein erfindungsgemäßes System mit einem erfindungsgemäßen Küchengerät in einem weiteren Ausführungsbeispiel.

In den nachfolgenden Figuren werden für die gleichen technischen Merkmale, auch von unterschiedlichen Ausführungsbeispielen, die identischen Bezugszeichen verwendet.

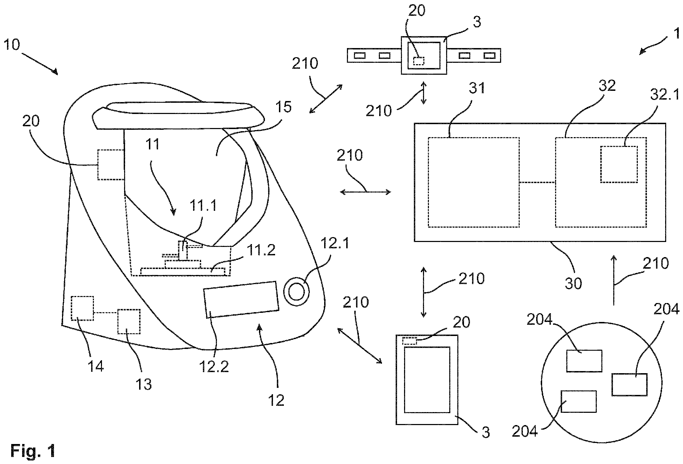

Figur 1 zeigt ein erfindungsgemäßes System 1 zum Zubereiten von Speisen in einem ersten Ausführungsbeispiel. Dabei weist das System 1 ein erfindungsgemäßes Küchengerät 10 zum zumindest teilweise Automatisieren und Abarbeiten eines Rezeptes auf. Dazu weist das Küchengerät 10 ferner eine Funktionseinheit 11 auf, die ein Rührwerk 11.1 und ein Heizelement 11.2 umfasst. Vorzugsweise ist die Funktionseinheit 11 in einem Kochgefäß 15 des Küchengerätes 10 angeordnet, sodass ein Benutzer Zutaten in das Kochgefäß 15 einfüllen kann und anschließend die Funktionseinheit 11 über eine Benutzerschnittstelle 12 des Küchengerätes 10 ansteuern kann. Dazu kann bspw. vorgesehen sein, dass ein Rezept auf einem Display 12.2 der Benutzerschnittstelle 12 durch ein Bedienelement 12.1 auswählbar und startbar ist. Durch das Rührwerk 11.1 können dann die Zutaten im Kochgefäß 15 zerkleinert werden und durch das Heizelement 11.2 erhitzt werden. Weiterhin weist das Küchengerät 10 eine Geräteschnittstelle 14 auf, durch welche das Küchengerät 10 mit einem Server 30 und/oder einem Nutzergerät 3 in Kommunikationsverbindung bringbar ist. Dazu kann das Küchengerät 10 über die Geräteschnittstelle 14 bspw. mit dem Internet verbindbar sein. Weiterhin weist das Küchengerät 10 ein integriertes Steuergerät 13 auf, durch welches ein erfindungsgemäßes Verfahren 100 zumindest initiierbar ist. Dabei kann eine Recheneinheit 31 des Servers 30 durch das Steuergerät 13 angesteuert werden, um das Verfahren 100 auszuführen.

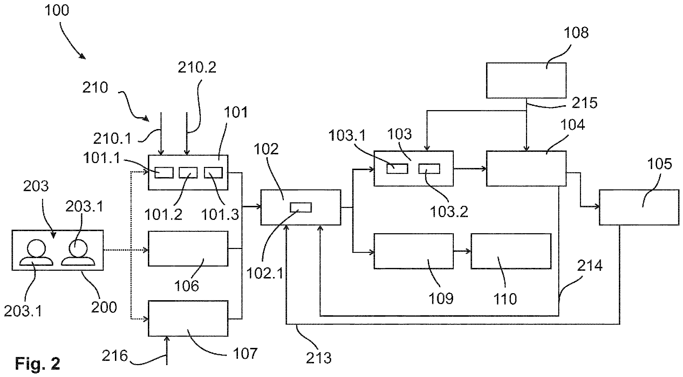

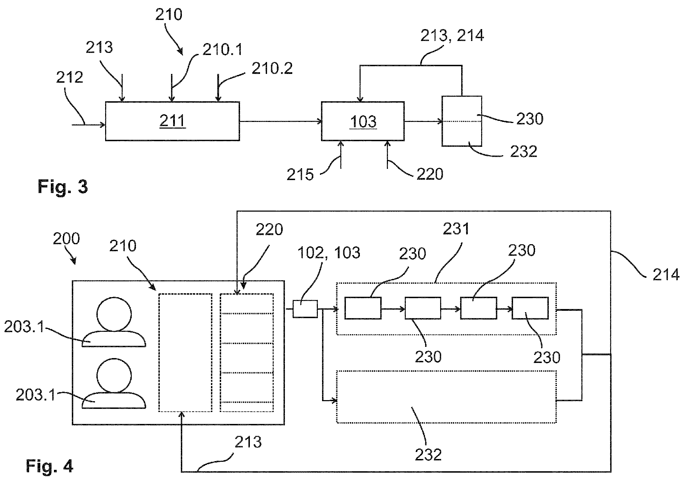

Das Verfahren 100 ist dabei in Figur 2 dargestellt. Dabei ist ein Erhalt 101 von nutzerspezifischen Gesundheitsdaten 210 vorgesehen, die einem Benutzerprofil 200 zuordbar sind. Insbesondere können die Gesundheitsdaten 210 dem Benutzerprofil 200 zugeordnet werden, wenn dieses aufgerufen wird. Der Aufruf des Benutzerprofils 200 kann z.B. die Eingabe von Login Daten durch den Benutzer, insbesondere am Küchengerät 10, erfordern. Dadurch kann sichergestellt sein, dass nur der Benutzer auf seine eigenen Daten zugreifen kann. Ferner ist es denkbar, dass das Benutzerprofil 200 eine Benutzergruppe 203 mit mehreren Einzelnutzern 203.1 umfasst. Dadurch kann z.B. ein Haushalt durch das Benutzerprofil 200 abgebildet sein. Die Gesundheitsdaten 210 können vorzugsweise Vitaldaten 210.1 und Aktivitätsdaten 210.2 umfassen. Die Vitaldaten 210.1 des Benutzers können z.B. medizinische Messwerte umfassen, wie einen Blutzuckerspiegel, eine durchschnittliche Pulsfrequenz und/oder dergleichen. Dazu kann ein Erfassen 101.3 der Aktivitätsdaten 210.2, z.B. in Form einer Häufigkeit sportlicher Aktivitäten, von Belastungen und/oder Schlafenszeiten, vorgesehen sein. Insbesondere können die Gesundheitsdaten 210, wie in Figur 1 dargestellt, durch ein Nutzergerät 3, vorzugsweise in Form eines Fitnessarmbandes, eines Smartphones oder Tablets, erhoben und/oder zur Verfügung gestellt werden. Der Erhalt 101 der Gesundheitsdaten 210 kann über eine Sensoreinheit 20 erfolgen, die Teil des Nutzergerätes 3 und/oder des Küchengerätes 10 ist. Die Sensoreinheit 20 kann zur Aufnahme der Gesundheitsdaten 210 z.B. eine Kamera, einen GPS-Sensor, eine Waage, einen Herzfrequenzsensor, einen Blutzuckersensor, einen Pulssensor und/oder einen Körperfettsensor umfassen. Insbesondere kann das Nutzergerät 3 selbst die Sensorvorrichtung 20 bilden. Ferner ist es denkbar, dass das Nutzergerät 3 mit dem Server 30 kommuniziert und die Gesundheitsdaten 210 über den Server 30 dem Küchengerät 10 zur Verfügung gestellt werden oder auf dem Küchengerät 10 verbleiben, um das Verfahren 100 weiter durchzuführen. Ferner kann vorgesehen sein, dass ein Auswerten 106 von Historiendaten 220 durchgeführt wird, wobei die Historiendaten 220 z.B. durchgeführte Kochvorgänge und/oder zuvor verwendetet Lebensmittel umfassen können. Dadurch kann neben den aktuellen Gesundheitsdaten 210 ein Rückschluss auf die Ernährung des Benutzers ermöglicht sein, wobei die Historiendaten 220 mit den nutzerspezifischen Gesundheitsdaten 210 bei einem Erstellen 102 einer Auswertung 211 zumindest der Gesundheitsdaten 210 für das Benutzerprofil 200 in Korrelation gebracht werden können. Die Auswertung 211 ist ferner in Figur 3 nochmal dargestellt, wobei bei der Auswertung 211 Gesundheitsdaten 210 in Form von Vitaldaten 210.1 und/oder Aktivitätsdaten 210.2 verwendet werden. Ferner können z.B. die Vitalveränderung 213 und Referenzdaten 212 bei der Auswertung 211 berücksichtigt werden. Zusätzlich kann ein Ermitteln 107 von Positionsinformationen 216 vorgesehen sein, die ebenfalls in die Auswertung 211 einfließen können. Die Positionsinformationen 216 können einen Aufenthaltsort des Benutzers in einer bestimmten Region aufzeigen, sodass regionale Einflüsse auf die Gesundheit berücksichtigt werden können. Ferner können die Positionsinformationen 216 Teil der Aktivitätsdaten 210.2. sein. Vorzugsweise findet im Rahmen der Auswertung 211 ein Vergleich 102.1 der Gesundheitsdaten 210 mit Referenzdaten 212 statt. Die Referenzdaten 212 können z.B. Idealdaten umfassen, die bei einem Benutzer desselben Alters, derselben Berufsgruppe und/oder dergleichen vorliegen sollten. Dadurch kann sich dementsprechend eine Differenz zwischen den vorliegenden Gesundheitsdaten 210 und den Referenzdaten 212 ergeben, die einen zu optimierenden Gesundheitszustand darstellt.

Basierend auf der Auswertung 211 erfolgt ein Bestimmen 103 einer Rezeptempfehlung 230, wobei vorzugsweise eine Auswahl 103.1 eines Rezeptes aus einer Rezeptdatenbank 32.1 erfolgt und/oder eine Generierung 103.2 eines Rezeptes anhand der Auswertung 211. Vorzugsweise können die Historiendaten 220 bei der Generierung 103.2 des Rezeptes berücksichtigt werden. Die Rezeptdatenbank 32.1 kann vorzugsweise Teil einer Speichereinheit 32 des Servers 30 sein. Anschließend erfolgt eine Ausgabe 104 der Rezeptempfehlung 230 an der Benutzerschnittstelle 12 des Küchengerätes 10, sodass der Benutzer die Rezeptempfehlung 230 unmittelbar am Küchengerät 10 nutzen kann, wenn er der Rezeptempfehlung 230 folgen und die entsprechende Mahlzeit am Küchengerät 10 vorbereiten möchte. Folglich kann insbesondere in Abhängigkeit von einer Benutzerreaktion 214 ein zumindest teilweise automatisiertes Abarbeiten 105 der Rezeptempfehlung 230 durch das Küchengerät 10 erfolgen. Die Benutzerreaktion 214 kann z.B. eine Bestätigung oder ein Ablehnen der Rezeptempfehlung 230 umfassen. Die positive oder negative Benutzerreaktion 214 kann für die nächste Auswertung 211 im Rahmen der Referenzdaten 212 und/oder der Historiendaten 220 genutzt werden, um die Präferenzen des Benutzers berücksichtigen zu können. Erfolgt ein Abarbeiten 105 der Rezeptempfehlung 230 kann ferner vorgesehen sein, dass eine Vitalreaktion 213 des Benutzers gemessen wird und ebenfalls bei der nächsten Auswertung 211 berücksichtigt wird. So ist es z.B. denkbar, dass auf Grundlage der Rezeptempfehlung 230 der Blutzuckerspiegel des Benutzers in einem bestimmten Maße steigt und somit bei der nächsten Auswertung 211 bekannt ist, inwiefern sich die Gesundheitsdaten 210 in Abhängigkeit von bestimmten Speisen bei dem Benutzer individuell auswirken. Um die Rezeptempfehlung 230 weiter zu verbessern, kann ein Erhalt 108 eines Benutzerziels 15 vorgesehen sein, das dem Benutzerprofil 200 zugeordnet ist oder zuordbar ist. Insbesondere kann der Benutzer das Benutzerziel 215 z.B. über das Nutzergerät 3 oder die Benutzerschnittstelle 12 dem Benutzerprofil 200 hinzufügen, sodass das Benutzerziel 215 beim Bestimmen 103 der Rezeptempfehlung 230 berücksichtigt wird. Das Benutzerziel 215 kann z.B. ein bestimmtes Fitnesslevel und/oder dergleichen umfassen. Auf Grundlage der Auswertung 211 kann ferner eine Aktivitätsempfehlung 232 gemäß Schritt 109 bestimmt werden und eine Ausgabe 110 der Aktivitätsempfehlung 232 am Nutzergerät 3 und/oder an der Benutzerschnittstelle 12 des Küchengerätes 10 erfolgen. Auch auf die Aktivitätsempfehlung 232 kann eine Benutzerreaktion 214 und/oder eine Vitalveränderung 213 erfolgen und bei der nächsten Auswertung 211 berücksichtigt werden.

Figur 4 zeigt eine detaillierte Darstellung des Benutzerprofils 200, welchem die nutzerspezifischen Gesundheitsdaten 210 sowie die Historiendaten 220 zugeordnet sind. Diese können mit dem Verfahren 100 verarbeitet werden, um zumindest eine Rezeptempfehlung 230 und vorzugsweise eine Aktivitätsempfehlung 232 zu erzeugen, wie zuvor beschrieben. Insbesondere kann vorgesehen sein, dass mehrere Rezeptempfehlungen 230 bestimmt werden und anhand der Rezeptempfehlungen 230 eine Rezeptfolge 231 erstellt wird. Die Rezeptfolge 231 kann dem Benutzerprofil 200 insbesondere in Abhängigkeit von der Benutzerreaktion 214 und/oder der Vitalveränderung 213 zugeordnet werden. Dadurch kann der Benutzer z.B. mehrere Rezeptempfehlungen 230 nacheinander abarbeiten und der langfristige Einfluss der Ernährungssteuerung Eingang in die Auswertung 211 finden.

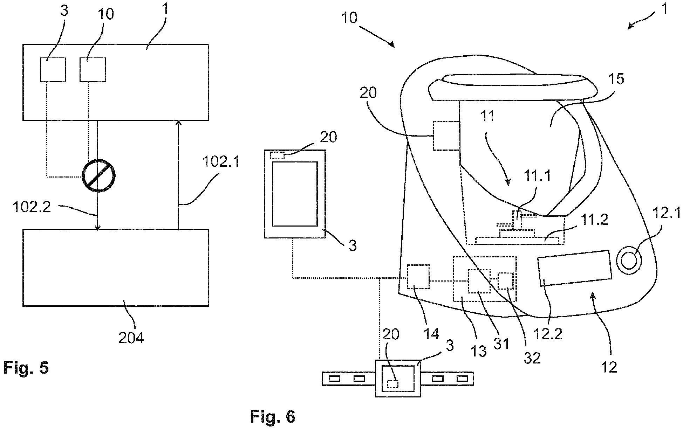

Figur 5 zeigt ferner eine Interaktion des Systems 1 mit einem externen Dienstleister 204, wobei ein externes Empfangen 101.1 von Gesundheitsdaten 210 durch die Geräteschnittstelle 14 des Küchengerätes 10 möglich ist und ein externes Versenden 101.2 der Gesundheitsdaten 210 zumindest teilweise verhindert ist. Dazu kann der Benutzer über die Benutzerschnittstelle 12 und/oder das Nutzergerät 3 das externe Versenden 101.2 der Gesundheitsdaten 210 an den externen Dienstleister 204 freischalten oder selektiv freischalten. Dadurch kann der externe Dienstleister 204, d.h. bspw. ein Fitnessstudio oder ein Arzt, Gesundheitsdaten 210 zur Verfügung stellen, um dadurch die Ernährungssteuerung mit dem Küchengerät 10 zu verbessern. Gleichzeitig ist dadurch ein Datenschutz gewährleistet, um insbesondere die Gesundheitsdaten 210 als besonders sensible persönliche Daten zu schützen.

Figur 6 zeigt ferner ein erfindungsgemäßes System 1 mit einem erfindungsgemäßen Küchengerät 10 in einem weiteren Ausführungsbeispiel. Dabei entspricht das System 1 im Wesentlichen dem System 1 des ersten Ausführungsbeispiels, wobei ein integriertes Steuergerät 13 des Küchengerätes 10 eine Recheneinheit 31 und eine Speichereinheit 32 aufweist. Insbesondere ist das erfindungsgemäße Verfahren 100 vollständig auf dem Küchengerät 10 ausführbar. Nutzergeräte 3 in Form eines Fitnessarmbandes und/oder eines Smartphones oder Tablets oder dergleichen können ferner über eine Geräteschnittstelle 14 mit dem Küchengerät 10 verbindbar sein, um Gesundheitsdaten 210 mit dem Küchengerät 10 auszutauschen.

### Bezugszeichenliste

- 1: System
- 3: Nutzergerät
- 10: Küchengerät
- 11: Funktionseinheit
- 11.1: Rührwerk
- 11.2: Heizelement
- 12: Benutzerschnittstelle
- 12.1: Bedienknopf
- 12.2: Display
- 13: integriertes Steuergerät
- 14: Geräteschnittstelle
- 15: Kochgefäß
- 20: Sensoreinheit
- 30: Server
- 31: Recheneinheit
- 32: Speichereinheit
- 32.1: Rezeptdatenbank
- 100: Verfahren
- 101: Erhalt von 210
- 101.1: externes Empfangen
- 101.2: externes Versenden
- 101.3: Erfassen von 210.2
- 102: Erstellen von 211
- 102.1: Vergleich von 210 und 212
- 103: Bestimmen von 230
- 103.1: Auswahl eines Rezeptes
- 103.2: Generierung eines Rezeptes
- 104: Ausgabe von 230
- 105: Abarbeiten von 230
- 106: Auswerten von 220
- 107: Ermitteln von Positionsinformationen
- 108: Erhalt von 215
- 109: Bestimmen von 232
- 110: Ausgabe von 232
- 200: Benutzerprofil
- 203: Benutzergruppe
- 203.1: Einzelnutzer von 203
- 204: Externer Dienstleister
- 210: Gesundheitsdaten
- 210.1: Vitaldaten
- 210.2: Aktivitätsdaten
- 211: Auswertung
- 212: Referenzdaten
- 213: Vitalveränderung
- 214: Benutzerreaktion
- 215: Benutzerziel
- 216: Positionsinformationen
- 220: Historiendaten
- 230: Rezeptempfehlung
- 231: Rezeptfolge
- 232: Aktivitätsempfehlung

## Patentansprüche

1. Verfahren (100) zur Ernährungssteuerung mit einem Küchengerät (10) zum zumindest teilweise automatisierten Abarbeiten eines Rezeptes umfassend die folgenden Schritte:
- Erhalt (101) von nutzerspezifischen Gesundheitsdaten (210), die einem Benutzerprofil (200) zuordbar sind,
- Erstellen (102) einer Auswertung (211) der Gesundheitsdaten (210) für das Benutzerprofil (200) durch eine Recheneinheit (31),
- Bestimmen (103) einer Rezeptempfehlung (230) auf Grundlage der Auswertung (211) der Gesundheitsdaten (210),
- Ausgabe (104) der Rezeptempfehlung (230) an einer Benutzerschnittstelle (12) des Küchengerätes (10),
**dadurch gekennzeichnet,**
**dass** beim Erstellen (102) der Auswertung (211) eine Benutzerreaktion (214) und eine Vitalveränderung (213) auf eine frühere Rezeptempfehlung (230) berücksichtigt wird, wodurch für die Auswertung (213) Randbedingungen in Form der Benutzerreaktion (214) und der Vitalveränderung (213) gesetzt werden, wobei die Gesundheitsdaten (210) Vitaldaten (210.1) eines Benutzers umfassen und/oder das Erstellen (102) der Auswertung (211) einen Vergleich (102.1) der Gesundheitsdaten (210) mit Referenzdaten (212) umfasst.

2. Verfahren (100) nach Anspruch 1,
**dadurch gekennzeichnet,**
**dass** das Verfahren (100) folgenden Schritt umfasst:
- Auswerten (106) von Historiendaten (220) zuvor durchgeführter Kochvorgänge und/oder zuvor verwendeter Lebensmittel,
wobei die Historiendaten (220) beim Erstellen (103) der Rezeptempfehlung (230) berücksichtigt werden.

3. Verfahren (100) nach einem der vorhergehenden Ansprüche,
**dadurch gekennzeichnet,**
**dass** das Verfahren (100) folgenden Schritt umfasst:
- Ermitteln (107) von Positionsinformationen (216) des Küchengerätes (10) und/oder eines Benutzers,
wobei die Positionsinformationen (216) beim Erstellen (103) der Rezeptempfehlung (230) berücksichtigt werden
und/oder dass das Verfahren (100) folgenden Schritt umfasst:
- Zumindest teilweise automatisiertes Abarbeiten (105) der Rezeptempfehlung (230) durch das Küchengerät (10).

4. Verfahren (100) nach einem der vorhergehenden Ansprüche,
**dadurch gekennzeichnet,**
**dass** das Bestimmen (103) der Rezeptempfehlung (230) zumindest einen der folgenden Schritte umfasst:
- Auswahl (103.1) eines Rezeptes aus einer Rezeptdatenbank (32.1) anhand der Auswertung (211) der Gesundheitsdaten (210), eines Benutzerziels (215) und/oder einer Vitalveränderung (213),
- Generierung (103.2) eines Rezeptes anhand der Auswertung (211) der Gesundheitsdaten (210) eines Benutzerziels (215) und/oder einer Vitalveränderung (213), insbesondere wobei die Historiendaten (220) bei der Generierung (103.2) des Rezeptes berücksichtigt werden.

5. Verfahren (100) nach einem der vorhergehenden Ansprüche,
**dadurch gekennzeichnet,**
**dass** ein externes Empfangen (101.1) der Gesundheitsdaten (210) durch die Geräteschnittstelle (14) möglich ist und ein externes Versenden (101.2) der Gesundheitsdaten (210) zumindest teilweise verhindert ist, insbesondere wobei das externe Versenden (101.2) der Gesundheitsdaten (210) durch den Benutzer freischaltbar oder selektiv freischaltbar ist.

6. Verfahren (100) nach einem der vorhergehenden Ansprüche,
**dadurch gekennzeichnet,**
**dass** die Gesundheitsdaten (210) Aktivitätsdaten (210.2) eines Benutzers umfassen und der Erhalt (101) der Gesundheitsdaten (210) folgenden Schritt umfasst: Erfassen (101.3) von Aktivitätsdaten (210.2).

7. Verfahren (100) nach einem der vorhergehenden Ansprüche,
**dadurch gekennzeichnet,**
**dass** der Erhalt (101) der Gesundheitsdaten (210) über eine Sensoreinheit (20) erfolgt, insbesondere wobei die Sensoreinheit (20) ein Nutzergerät (3) umfasst und/oder einen der folgenden Sensoren umfasst:
- Kamera,
- GPS-Sensor,
- Waage,
- Temperatursensor (insbesondere berührungslos)
- Herzfrequenzsensor,
- Blutzuckersensor,
- Blutdrucksensor,
- Pulssensor,
- Körperfettsensor.

8. Verfahren (100) nach einem der vorhergehenden Ansprüche,
**dadurch gekennzeichnet,**
**dass** das Verfahren (100) folgenden Schritt umfasst:
- Erhalt (108) eines Benutzerziels (215), das dem Benutzerprofil (200) zuordbar ist, wobei das Benutzerziel (215) beim Bestimmen (103) der Rezeptempfehlung (230) berücksichtigt wird.

9. Verfahren (100) nach einem der vorhergehenden Ansprüche,
**dadurch gekennzeichnet,**
**dass** in Abhängigkeit von den Gesundheitsdaten (210), den Positionsinformationen (216) und/oder dem Benutzerziel (215) eine Rezeptfolge (231) bestimmt wird und/oder dass die Rezeptfolge (231) in Abhängigkeit von der Benutzerreaktion (214) und/oder der Vitalveränderung (213) auf die Rezeptempfehlung (230) der Rezeptfolge (231) modifiziert wird.

10. Verfahren (100) nach einem der vorhergehenden Ansprüche,
**dadurch gekennzeichnet,**
**dass** dem Benutzerprofil (200) eine Benutzergruppe (203), insbesondere ein Haushalt, zugeordnet wird.

11. Verfahren (100) nach einem der vorhergehenden Ansprüche,
**dadurch gekennzeichnet,**
**dass** das Verfahren (100) folgende Schritte umfasst:
- Bestimmen (109) einer Aktivitätsempfehlung (232) auf Grundlage der Auswertung (211) der Gesundheitsdaten (210),
- Ausgabe (110) der Aktivitätsempfehlung (232), insbesondere an der Benutzerschnittstelle (12) des Küchengerätes (10).

12. Küchengerät (10) zum zumindest teilweise automatisierten Abarbeiten eines Rezeptes aufweisend
eine Funktionseinheit (11) zum Bearbeiten von Zutaten, und
eine Benutzerschnittstelle (12) für eine Interaktion mit einem Benutzer,
**dadurch gekennzeichnet,**
**dass** die Benutzerschnittstelle (12) mit einem integrierten Steuergerät (13) verbunden ist, wobei das Steuergerät (13) eine Recheneinheit (31) und eine Speichereinheit (32) aufweist, durch welches ein Verfahren (100) nach einem der vorhergehenden Ansprüche durchführbar ist.

13. System (1) zum Zubereiten von Speisen aufweisend
ein Küchengerät (10), insbesondere nach dem vorhergehenden Anspruch, zum zumindest teilweise automatisierten Abarbeiten eines Rezeptes mit einer Funktionseinheit (11) zum Bearbeiten von Zutaten und einer Benutzerschnittstelle (12) für eine Interaktion mit einem Benutzer, und
eine Speichereinheit (32), in welcher ein Benutzerprofil (200) und nutzerspezifische Gesundheitsdaten (210) abspeicherbar sind,
wobei eine Recheneinheit (31) vorgesehen ist, die dazu ausgebildet ist eine Auswertung (211) der Gesundheitsdaten (210) zu erstellen und in Abhängigkeit der Auswertung (211) eine Rezeptempfehlung (230) zu bestimmen und an der Benutzerschnittstelle (12) auszugeben, wobei die Recheneinheit (31) dazu ausgebildet ist, ein Verfahren (100) nach einem der Ansprüche 1 bis 11 durchzuführen.

14. System (1) nach Anspruch 13,
**dadurch gekennzeichnet,**
**dass** die Recheneinheit (31) und/oder die Speichereinheit (32) zur Erhebung von nutzerspezifischen Gesundheitsdaten (210) mit einem weiteren Küchengerät (10) und/oder einem Nutzergerät (3) in Kommunikationsverbindung bringbar ist
und/oder dass ein Server (30) die Recheneinheit (31) und/oder die Speichereinheit (32) umfasst.

15. System (1) nach einem der vorhergehenden Ansprüche,
**dadurch gekennzeichnet,**
**dass** das Küchengerät (10) eine Geräteschnittstelle (14) aufweist, durch welche das Küchengerät (10) mit der Recheneinheit (31) und/oder der Speichereinheit (32), insbesondere zum Zugriff auf eine Rezeptdatenbank (32.1), in Kommunikationsverbindung bringbar ist.

## Claims

1. A method (100) of food control with a kitchen appliance (10) for at least partially automating the processing of a recipe comprising the following stages:
- Receiving (101) user-specific health data (210) that can be assigned to a user profile (200),
- Creating (102) an evaluation (211) of the health data (210) for the user profile (200) by a computing unit (31),
- Determining (103) a recipe recommendation (230) based on the evaluation (211) of the health data (210),
- Output (104) of the recipe recommendation (230) at a user interface (12) of the kitchen appliance (10),
**characterized in that**
a user reaction (214) and a vital change (213) to a previous recipe recommendation (230) are taken into account when creating (102) the evaluation (211), as a result of which boundary conditions in the form of the user reaction (214) and the vital change (213) are set for the evaluation (213), the health data (210) comprising vital data (210.1) of a user and/or creating (102) the evaluation (211) comprising a comparison (102.1) of the health data (210) with reference data (212).

2. The method (100) according to claim 1,
**characterized in that**
the method (100) comprises the following stage:
- Evaluation (106) of history data (220) of previously performed cooking processes and/or previously used foodstuffs,
wherein the history data (220) is taken into account when creating (103) the recipe recommendation (230).

3. The method (100) according to any one of the preceding claims,
**characterized in that**
the method (100) comprises the following stage:
- Determining (107) position information (216) of the kitchen appliance (10) and/or a user,
wherein the position information (216) is taken into account when generating (103) the recipe recommendation (230)
and/or **in that** the method (100) comprises the following stage:
- At least partially automated processing (105) of the recipe recommendation (230) by the kitchen appliance (10).

4. The method (100) according to any one of the preceding claims,
**characterized in that**
determining (103) of the recipe recommendation (230) comprises at least one of the following stages:
- Selection (103.1) of a prescription from a prescription database (32.1) based on the evaluation (211) of the health data (210), a user goal (215) and/or a vital change (213),
- Generation (103.2) of a prescription on the basis of the evaluation (211) of the health data (210) of a user target (215) and/or a vital change (213), in particular wherein the history data (220) are taken into account in the generation (103.2) of the prescription.

5. The method (100) according to any one of the preceding claims,
**characterized in that**
the external reception (101.1) of the health data (210) by the device interface (14) is possible and external transmission (101.2) of the health data (210) is at least partially prevented, in particular wherein the external transmission (101.2) of the health data (210) can be enabled or selectively enabled by the user.

6. The method (100) according to any one of the preceding claims,
**characterized in that**
the health data (210) comprises activity data (210.2) of a user, and the receiving (101) of the health data (210) comprises the following stage:
Recording (101.3) of activity data (210.2).

7. The method (100) according to any one of the preceding claims,
**characterized in that**
the health data (210) is received (101) via a sensor unit (20),
in particular wherein the sensor unit (20) comprises a user device (3) and/or comprises one of the following sensors:
- Camera,
- GPS sensor,
- Libra,
- Temperature sensor (especially non-contact)
- Heart rate sensor,
- Blood glucose sensor,
- Blood pressure sensor,
- Pulse sensor,
- Body fat sensor.

8. The method (100) according to any one of the preceding claims,
**characterized in that**
the method (100) comprises the following stage:
- Obtaining (108) a user goal (215) that can be assigned to the user profile (200), wherein the user goal (215) is taken into account when determining (103) the recipe recommendation (230).

9. The method (100) according to any one of the preceding claims,
**characterized in that**
a recipe sequence (231) is determined as a function of the health data (210), the position information (216) and/or the user destination (215)
and/or that the recipe sequence (231) is modified depending on the user reaction (214) and/or the vital change (213) to the recipe recommendation (230) of the recipe sequence (231).

10. The method (100) according to any one of the preceding claims,
**characterized in that**
a user group (203), in particular a household, is assigned to the user profile (200).

11. The method (100) according to any one of the preceding claims,
**characterized in that**
the method (100) comprises the following stages:
- Determining (109) an activity recommendation (232) based on the evaluation (211) of the health data (210),
- Output (110) of the activity recommendation (232), in particular at the user interface (12) of the kitchen appliance (10).

12. Kitchen appliance (10) for at least partially automated processing of a recipe, comprising a functional unit (11) for processing ingredients, and
a user interface (12) for interaction with a user,
**characterized in that**
the user interface (12) is connected to an integrated control unit (13), the control unit (13) having a computing unit (31) and a memory unit (32), by means of which a method (100) according to one of the preceding claims can be carried out.

13. System (1) for preparing food, comprising
a kitchen appliance (10), in particular according to the preceding claim, for at least partially automated processing of a recipe with a functional unit (11) for processing ingredients and a user interface (12) for interaction with a user, and
a memory unit (32) in which a user profile (200) and user-specific health data (210) can be stored,
wherein a computing unit (31) is provided, which is designed to create an evaluation (211) of the health data (210) and to determine a recipe recommendation (230) as a function of the evaluation (211) and to output it at the user interface (12), wherein the computing unit (31) is designed to perform a method (100) according to one of claims 1 to 11.

14. System (1) according to claim 13,
**characterized in that**
the computing unit (31) and/or the memory unit (32) for collecting user-specific health data (210) can be brought into communication with a further kitchen appliance (10) and/or a user appliance (3)
and/or that a server (30) comprises the computing unit (31) and/or the memory unit (32).

15. System (1) according to one of the preceding claims,
**characterized in that**
the kitchen appliance (10) has an appliance interface (14) through which the kitchen appliance (10) can be brought into communication with the computing unit (31) and/or the memory unit (32), in particular for accessing a recipe database (32.1).

## Revendications

1. Procédé (100) de contrôle de l'alimentation avec un appareil de cuisine (10) pour le traitement au moins partiellement automatisé d'une recette, comprenant les étapes suivantes :
- L'obtention (101) de données de santé (210) spécifiques à l'utilisateur, qui peuvent être associées à un profil d'utilisateur (200),
- La création (102) d'une évaluation (211) des données de santé (210) pour le profil d'utilisateur (200) par une unité de calcul (31),
- Déterminer (103) une recommandation de recette (230) sur la base de l'évaluation (211) des données de santé (210),
- La sortie (104) de la recommandation de recette (230) sur une interface utilisateur (12) de l'appareil de cuisine (10),
**caractérisé en ce que**
lors de la création (102) de l'évaluation (211), on tient compte d'une réaction (214) de l'utilisateur et d'une modification vitale (213) à une recommandation de recette (230) antérieure, ce qui fixe pour l'évaluation (213) des conditions limites sous la forme de la réaction (214) de l'utilisateur et de la modification vitale (213), les données de santé (210) comprenant des données (210.1) vitales d'un utilisateur et/ou la création (102) de l'évaluation (211) comprenant une comparaison (102.1) des données (210) de santé à des données (212) de référence.

2. Procédé (100) selon la revendication 1,
**caractérisé en ce que**
le procédé (100) comprend l'étape suivante :
- Évaluer (106) des données d'historique (220) d'opérations de cuisson précédemment effectuées et/ou d'aliments précédemment utilisés,
dans lequel les données d'historique (220) sont prises en compte lors de la création (103) de la recommandation de recette (230).

3. Procédé (100) selon l'une quelconque des revendications précédentes,
**caractérisé en ce que**
le procédé (100) comprend l'étape suivante :
- Déterminer (107) des informations de position (216) de l'appareil de cuisine (10) et/ou d'un utilisateur,
dans lequel les informations de position (216) sont prises en compte lors de la création (103) de la recommandation de recette (230)
et/ou **en ce que** le procédé (100) comprend l'étape suivante :
- Traitement au moins partiellement automatisé (105) de la recommandation de recette (230) par l'appareil de cuisine (10).

4. Procédé (100) selon l'une quelconque des revendications précédentes,
**caractérisé en ce que**
la détermination (103) de la recommandation de recette (230) comprend au moins l'une des étapes suivantes :
- Sélection (103.1) d'une recette à partir d'une base de données de recettes (32.1) sur la base de l'évaluation (211) des données de santé (210), d'un objectif d'utilisateur (215) et/ou d'un modification vitale (213),
- Génération (103.2) d'une recette à l'aide de l'évaluation (211) des données de santé (210) d'un objectif d'utilisateur (215) et/ou d'une modification des modifications vitaux (213), en particulier les données historiques (220) étant prises en compte lors de la génération (103.2) de la recette.

5. Procédé (100) selon l'une quelconque des revendications précédentes,
**caractérisé en ce qu'**
une réception externe (101.1) des données de santé (210) est possible par l'interface d'appareil (14) et qu'un envoi externe (101.2) des données de santé (210) est au moins partiellement empêché, en particulier l'envoi externe (101.2) des données de santé (210) pouvant être débloqué ou débloqué sélectivement par l'utilisateur.

6. Procédé (100) selon l'une quelconque des revendications précédentes,
**caractérisé en ce que**
les données de santé (210) comprennent des données d'activité (210.2) d'un utilisateur et **en ce que** la réception (101) des données de santé (210) comprend l'étape suivante :
Saisie (101.3) de données d'activité (210.2).

7. Procédé (100) selon l'une quelconque des revendications précédentes,
**caractérisé en ce que**
la réception (101) des données de santé (210) s'effectue par l'intermédiaire d'une unité de capteur (20),
en particulier dans lequel l'unité de capteurs (20) comprend un appareil utilisateur (3) et/ou comprend l'un des capteurs suivants :
- Caméra,
- Capteur GPS,
- Balance,
- Capteur de température (en particulier sans contact)
- Capteur de fréquence cardiaque,
- Capteur de glycémie,
- Capteur de pression artérielle,
- Capteur de pouls,
- Capteur de graisse corporelle.

8. Procédé (100) selon l'une quelconque des revendications précédentes,
**caractérisé en ce que**
le procédé (100) comprend l'étape suivante :
- L'obtention (108) d'un objectif de l'utilisateur (215) qui peut être associée au profil d'utilisateur (200),
dans lequel l'objectif de l'utilisateur (215) est pris en compte lors de la détermination (103) de la recommandation de recette (230).

9. Procédé (100) selon l'une quelconque des revendications précédentes,
**caractérisé en ce qu'**
en fonction des données de santé (210), des informations de position (216) et/ou de la destination de l'utilisateur (215), une séquence de recettes (231) est déterminée
et/ou **en ce que** la séquence de recettes (231) est modifiée en fonction de la réaction de l'utilisateur (214) et/ou du changement des modification vitaux (213) à la recommandation de recette (230) de la séquence de recettes (231).

10. Procédé (100) selon l'une quelconque des revendications précédentes,
**caractérisé en ce qu'**
un groupe d'utilisateurs (203), en particulier un ménage, est associé au profil d'utilisateur (200).

11. Procédé (100) selon l'une quelconque des revendications précédentes,
**caractérisé en ce que**
le procédé (100) comprend les étapes suivantes :
- détermination (109) d'une recommandation d'activité (232) sur la base de l'évaluation (211) des données de santé (210),
- sortie (110) de la recommandation d'activité (232), en particulier sur l'interface utilisateur (12) de l'appareil de cuisine (10).

12. Appareil de cuisine (10) pour le traitement au moins partiellement automatisé d'une recette, présentant
une unité fonctionnelle (11) pour le traitement d'ingrédients, et
une interface utilisateur (12) pour une interaction avec un utilisateur,
**caractérisé en ce que**
l'interface utilisateur (12) est reliée à un appareil de commande intégré (13), l'appareil de commande (13) présentant une unité de calcul (31) et une unité de mémoire (32), par lequel un procédé (100) selon l'une des revendications précédentes peut être mis en oeuvre.

13. Système (1) pour la préparation d'aliments, comprenant
un appareil de cuisine (10), en particulier selon la revendication précédente, pour le traitement au moins partiellement automatisé d'une recette avec une unité fonctionnelle (11) pour le traitement d'ingrédients et une interface utilisateur (12) pour une interaction avec un utilisateur, et
une unité de mémoire (32) dans laquelle un profil d'utilisateur (200) et des données de santé (210) spécifiques à l'utilisateur peuvent être stockés,
une unité de calcul (31) étant prévue, laquelle est conçue pour établir une évaluation (211) des données de santé (210) et pour déterminer une recommandation de recette (230) en fonction de l'évaluation (211) et la délivrer à l'interface utilisateur (12), l'unité de calcul (31) étant conçue pour exécuter un procédé (100) selon l'une des revendications 1 à 11.

14. Système (1) selon la revendication 13,
**caractérisé en ce que**
l'unité de calcul (31) et/ou l'unité de mémoire (32) peut être mise en liaison de communication avec un autre appareil de cuisine (10) et/ou un appareil d'utilisateur (3) pour la collecte de données de santé (210) spécifiques à l'utilisateur
et/ou **en ce qu'**un serveur (30) comprend l'unité de calcul (31) et/ou l'unité de mémoire (32).

15. Système (1) selon l'une des revendications précédentes,
**caractérisé en ce que**
l'appareil de cuisine (10) présente une interface d'appareil (14) par laquelle l'appareil de cuisine (10) peut être mis en liaison de communication avec l'unité de calcul (31) et/ou l'unité de mémoire (32), en particulier pour accéder à une base de données de recettes (32.1).
